# EUROPEAN PATENT APPLICATION

(11) **EP 2 535 008 A2**
(43) Date of publication of application: **19.12.2012**
(21) Application number: 11740049.9
(22) Date of filing: 07.02.2011
(51) Int. Cl.: A61B 17/12, A61B 17/122, A61L 31/02, A61L 17/00

(54) **EMBOLUS-FORMING IN-VIVO INDWELLING COIL AND METHOD FOR MANUFACTURING AN EMBOLUS-FORMING IN-VIVO INDWELLING COIL**

(30) Priority: 27.12.2010 KR 20100135692; 08.02.2010 KR 20100011283
(71) Applicant: Taewoong Medical Co., Ltd., Kimpo-si, Kyunggi do 415-873 (KR); Kwon, O Ki, Gangnam-Gu, Seoul 135-280 (KR)
(72) Inventor: KWON, O KI, Seoul 135-280 (KR)
(74) Representative: Johnston, Magnus George
(86) International application number: PCT/KR2011/000764
(87) International publication number: WO 2011/096753

(57) **Abstract**

The present invention relates to an embolus-forming in-vivo indwelling coil in which the direction of action of force being applied to the embolus-forming coil changes by the curvature of the coil when the coil is in-vivo indwelled, such that the coil can be smoothly indwelled without strongly pressing the blood vessel wall of cerebral aneurysm and the coil can be tightly indwelled in the cerebral aneurysm. To accomplish this object, the embolus-forming in-vivo indwelling coil of the present invention has one or more curvatures formed on a unit helical coil of a secondary coil and the curvatures are connected to two angle-changing portions. A method for manufacturing the coil forms the above-described curvatures and the angle-changing portions for the curvatures.

## Description

### [Technical Field]

The present invention relates, in general, to an embolus-forming in-vivo indwelling coil, which is used in endovascular treatment, and more particularly, to an embolus-forming in-vivo indwelling coil, which can be filled at a high density inside a cerebral aneurysm while acting so that it can be smoothly indwelled without strongly pressing the wall of the blood vessel of the cerebral aneurysm when being brought into contact with the inner wall of a cerebral artery, and a method for manufacturing the embolus-forming in-vivo indwelling coil.

### [Background Art]

Cerebral aneurysm is a disease characterized by abnormal swelling of a cerebral artery, which has a high danger of rupture compared to the wall of a normal artery. When ruptured, the cerebral aneurysm causes severe cerebral hemorrhage, including bleeding.

As a cure for the cerebral aneurysm, it is typical to clip an aneurysm by craniotomy and insert a separable embolization coil into the cerebral aneurysm by approaching the aneurysm via a blood vessel. In this fashion, the cerebral aneurysm is removed from a blood flow, thereby preventing rebleeding.

The first purpose of the cure of the cerebral aneurysm is to prevent rebleeding owing to the rupture or rerupture of the aneurysm. A traditional cure thereof is to clip a cervical portion of the aneurysm by craniotomy.

The skull is opened, and a blood vessel which has the aneurysm is found. Then, the neck portion of the aneurysm is held by a clip made of a metal so that a blood flow does not enter the aneurysm.

With the development of the separable embolization coil, a narrow catheter is inserted into a blood vessel so that the leading end of the catheter is input into the aneurysm. A sufficient amount of the separable embolization coil is then inserted into the aneurysm through the catheter. Consequently, the aneurysm is filled with the coil so that no blood flow enters the aneurysm any more. As a result, a cure that prevents rebleeding due to the re-rupturing of the aneurysm is made possible.

Such a separable embolization coil has an advantage in that it can be separated from a coil pusher only when required. Therefore, it is possible to repeat inserting and removing the embolization coil, thereby forming an embolus in the aneurysm according to the shape of the aneurysm without blood flow disturbances in a patent artery.

The embolus-forming coil is introduced into the cerebral aneurysm through a suitable catheter by an extruding means (inducer), which is detachably connected to one end thereof.

Such an embolus-forming in-vivo indwelling coil is used for intravascular treatment of the cerebral aneurysm. Recently, intravascular treatment using the embolus-forming in-vivo indwelling coil is widely performed since it can evade craniotomy.

The embolus-forming in-vivo indwelling coil is divided depending on the shape thereof, and is used in surgical operations by being generally divided into a helical coil, which has a helical shape, and a complex coil, which has a complex shape.

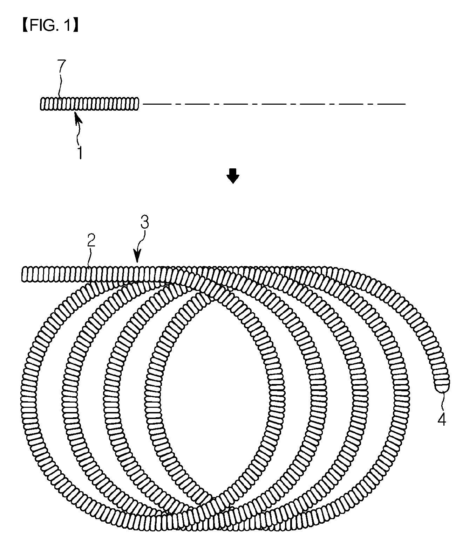

The helical coil 3 shown in accompanying FIG. 1 is produced by preparing a first coil by forming a wire 7 made of a material, such as platinum, silver, gold, palladium or tungsten, into a helical shape, heat-treating a first coil 1 such that it remembers the helical shape, forming the first coil into a helical shape again, and in this state, forming a secondary coil 2 by heat-treating the first coil 1 such that it remembers this helical shape. A chip 4 is provided on one end of the secondary coil 2 in order to prevent the inner wall of a blood vessel from being damaged.

Such a helical coil is installed by inserting it into a cerebral aneurysm through a catheter.

In addition, in the case of the helical coil 3 as described above, in order to prevent the cerebral aneurysm from being reopened, it is necessary to fill the inside of the cerebral aneurysm as tight as possible. However, the helical coil 3 as described above has a limited ability to tightly fill the inside of the cerebral aneurysm since it is wound only in one direction. In addition, since the helical coil is filled in the helical shape, the helical first coil 1 is filled while having the helical shape of the secondary coil 2 that remembers its shape. Consequently, force is not distributed and the coil is brought into contact with the inner wall of the blood vessel at the same force, which is problematic.

Because of this problem, provided is a complex coil 4, which has a complex shape in order to fill the inside of a cerebral aneurysm with the coil as tight as possible.

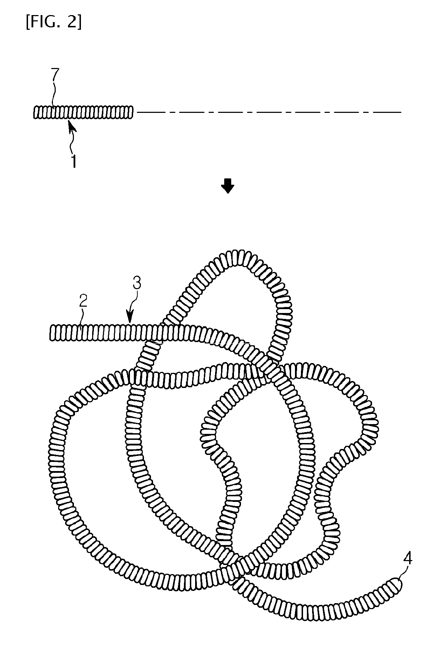

This is completed by preparing a first coil 1 such that it has a helical shape, heat-treating the first coil such that it remembers the helical shape, and heat-treating the first coil in the state that has a complex shape such that it forms a complex secondary coil 5, as shown in FIG. 2.

The advantage of the complex coil 4, which has the complex shape, is that it can more tightly fill the inside of the cerebral aneurysm than the helical coil 1.

In this case, the coil restores the remembered original shape, i.e. the shape of the complex secondary coil 5, while being discharged through the catheter and inserted into the endovascular aneurysm. However, during this process, it is impossible to predict the movement of the coil. In addition, as described above, the complex secondary coil 5 has a complicated configuration due to the helical shape thereof. Consequently, the coil fills the aneurysm while staying in the helical shape. Therefore, force is not distributed and the coil is brought into contact with the inner wall of the blood vessel under the same force, which is problematic.

That is, the above-described embolus-forming coil of the related art has the problem in that the force applied to the embolus-forming coil acts only in one direction when the coil is in-vivo indwelled. Because of this problem, it is difficult to securely indwell the coil in the cerebral aneurysm, which is heavily bent.

### [Disclosure]

### [Technical Problem]

Accordingly, the present invention has been made keeping in mind the above problems occurring in the related art, and is intended to provide an embolus-forming in-vivo indwelling coil in which the direction in which force applied to the embolus-forming coil acts is changed due to the curvature of the coil when the coil is in-vivo indwelled, so that the coil can be smoothly indwelled without strongly pressing the blood vessel wall of a cerebral aneurysm and the coil can be tightly indwelled in the cerebral aneurysm.

### [Technical Solution]

In order to realize the foregoing object, the present invention provides an embolus-forming in-vivo indwelling coil, which has one or more curvature portions on a unit helical coil of a secondary coil, with each of the curvature portions being connected to two angle-changing portions, and a manufacturing method for forming the curvature portions while forming the angle-changing portions, which are required for the formation of the curvature portions.

### [Advantageous Effects]

In the embolus-forming in-vivo indwelling coil of the invention, one or more curvature portions and two angle-changing portions, which are connected to a respective curvature portion, are formed in a unit helical coil of the secondary coil. When external force is applied to the embolus-forming coil when the embolus-forming indwelling coil is in-vivo indwelled, the angle-changing portions prevent the force from acting in only one direction but enable the pressing force to be distributed, so that the coil can be smoothly indwelled. When the embolus-forming indwelling coil is indwelled inside the cerebral aneurysm, the indwelling coil is indwelled also in the curvature portions. Consequently, it is possible to tightly indwell the indwelling coil.

### [Description of Drawings]

FIG. 1 is a reference view of a helical coil of the related art;
FIG. 2 is a reference view of a complex coil;

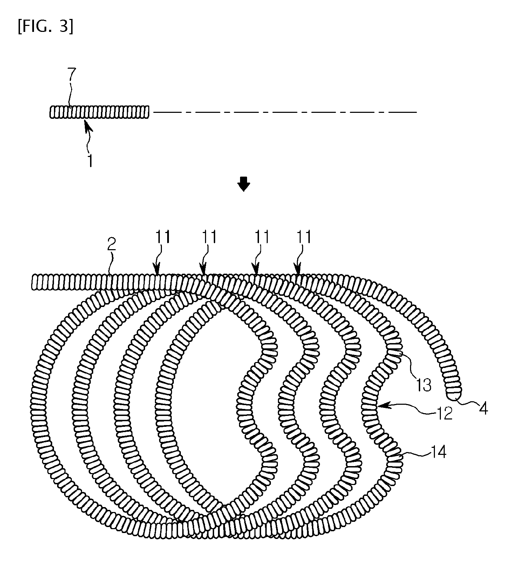

FIG. 3 is a reference view of the present invention;
FIG. 4 is a reference view showing key parts, which are extracted from FIG. 3;
FIG. 5 and FIG. 6 are views of other embodiments of the invention; and
FIG. 7 is a view of a process of fabricating a coil of the invention; and
FIG. 8 is a view of another process of fabricating a coil of the invention.

| | |
|---|---|
| 1: first coil | 2: secondary coil |
| 3: helical coil | 11: unit helical coil |
| 12: curvature portion | 13, 14: angle-changing portion |
| 20: circular rod | 21, 22: curved end portion |
| 23: curved recess | 24: jig |
| 25: pressing circular rod | |

### [Best Mode]

The present invention is intended to provide an embolus-forming in-vivo indwelling coil in which the direction in which force applied to the embolus-forming coil acts is changed owing to the curvature of the coil when the coil is in-vivo indwelled, so that the coil can be smoothly indwelled without strongly pressing the blood vessel wall of a cerebral aneurysm and the coil can be tightly indwelled in the cerebral aneurysm.

### [Mode for Invention]

Reference will now be made in greater detail to the present invention with reference to the accompanying drawings.

As in the related art, a helical coil 3 is produced by preparing a helical first coil 1 by forming a wire 7 made of a material, such as platinum, silver, gold, palladium or tungsten, into a helical shape, heat-treating the first coil 1 such that it remembers the helical shape, forming the first coil 1 into a helical shape again, and in this state, forming a secondary coil 2 by heat-treating the first coil 1 such that it remembers this helical shape.

In the helical coil, the present invention provides an embolus-forming in-vivo indwelling coil characterized in that the secondary coil 2 is composed of a plurality of unit helical coils 11, each of which has one or more curvature portions 12. Both ends of each curvature portion 12 are integrally connected with angle-changing portions 13 and 14, which are bent such that they are curved inwards.

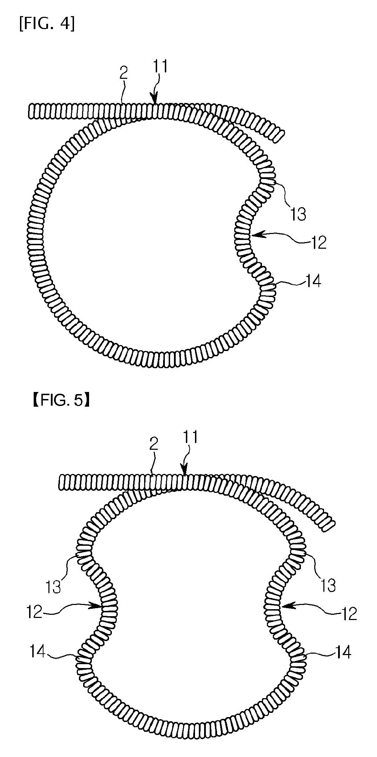

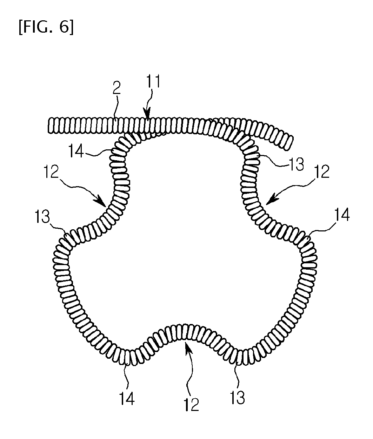

As shown in FIG. 5 and FIG. 6, the curvature portions 12 may be configured such that there are two or more of them.

In this case, when two curvature portions 12 are formed on each unit helical coil 11, the curvature portions are formed in opposite positions. When three or more curvature portions 12 are formed, it is preferred that the curvature portions be spaced apart by equal intervals. This is equivalent to saying that the angle-changing portions 13 and 14, which are formed for each unit curvature portion 12, be equally spaced apart from each other.

In this fashion, the curvature portions 12 are formed on each of the unit helical coil 11 of the secondary coil 2. The curvature portions 12 are bent from the circumference at the angle-changing portions 13 and 14 such that the curvature portions are curved inwards. In addition, the angle-changing portions 13 and 14 are shaped such that they are bent from the circumference so as to be curved inwards. Thus, in the process in which the embolus-forming in-vivo indwelling coil of the invention is indwelled in a cerebral aneurysm, when the indwelling coil comes into contact with any region of the wall of the blood vessel of the cerebral aneurysm, the angle-changing portions 13 and 14 are bent by the external force attributable to contact with the wall. Consequently, the indwelling coil does not proceed in one direction but changes the direction in which it proceeds, and the pressing force is distributed. As a result, the indwelling coil is smoothly indwelled inside the cerebral aneurysm.

In addition, since the coil is configured such that the curvature portions 12 are curved inwards in response to continued indwelling of the coil, the coil can also be indwelled in this region also. Consequently, the coil can be more tightly indwelled. In addition, the direction in which force is applied to the embolus-forming indwelling coil in the in-vivo indwelling of the coil changes in response to the bending of the cerebral aneurysm, so that the coil can be reliably indwelled at a position in the blood vessel.

In addition, when a plurality of bent portions 12 is provided in the above, it is preferred that the bent portions stay spaced apart by equal intervals on the unit helical coil 11. Because of this structure, when the indwelling coil is in-vivo indwelled, external force that is applied to each unit helical coil 11 is equally distributed by the angle-changing portions 13 and 14, which are integrally connected to the curvature portions 12 that have equal intervals, so that the in-vivo indwelling can be smoothly performed.

However, this merely illustrates an example in which it is preferred that the external force be equally distributed, since the force is distributed when the force is applied even though the intervals are not equal.

A description will be given of a method of manufacturing the embolus-forming in-vivo indwelling coil of the invention as described above.

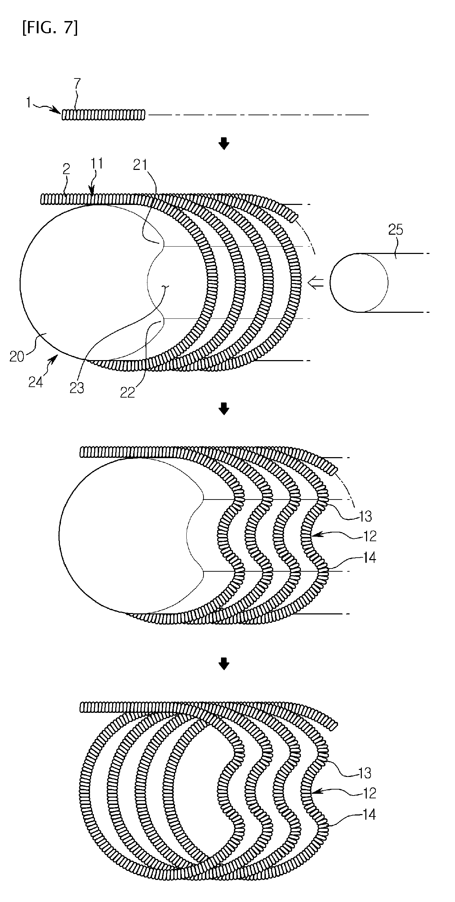

As shown in accompanying FIG. 7, the first coil 1, which is prepared by forming a wire 7 made of a material, such as platinum, silver, gold, palladium or tungsten, into a helical shape, is heat-treated such that the first coil remembers the helical shape.

When the object is to form the secondary coil in this state, a jig 24 is used. The jig 24 is shaped by forming curved end portions 21 and 22 and a curved recess 23 in a pressing circular rod 20 along the length thereof. The curved end portions 21 and 22 face each other, and the curved recess 23 is connected to the curved end portions 21 and 22.

In the state in which a required length of the first coil 1 is helically wound on the jig 24, predetermined portions of the first coil 1 that correspond to the curved recess 23 are pressed in the longitudinal direction using the pressing circular rod 25, and the pressed state is maintained.

In this state, the portions of the first coil 1 that are at positions that correspond to the curved recess 23 are inserted into the curved recess 23 by the pressing circular rod 25.

When the first coil is heat-treated such that it remembers this state, the embolus-forming in-vivo indwelling coil of the invention that the present invention is intended to provide is realized. Here, in the secondary coil 2, the curvature portions 12 having a curved shape are formed on each unit helical coil 11 by the curved recess 23. In addition, the angle-changing portions 13 and 14, which are bent such that they are curved inwards in order to form the curvature portions, are formed at both ends of the curvature portions 12 by the curved ends 21 and 22.

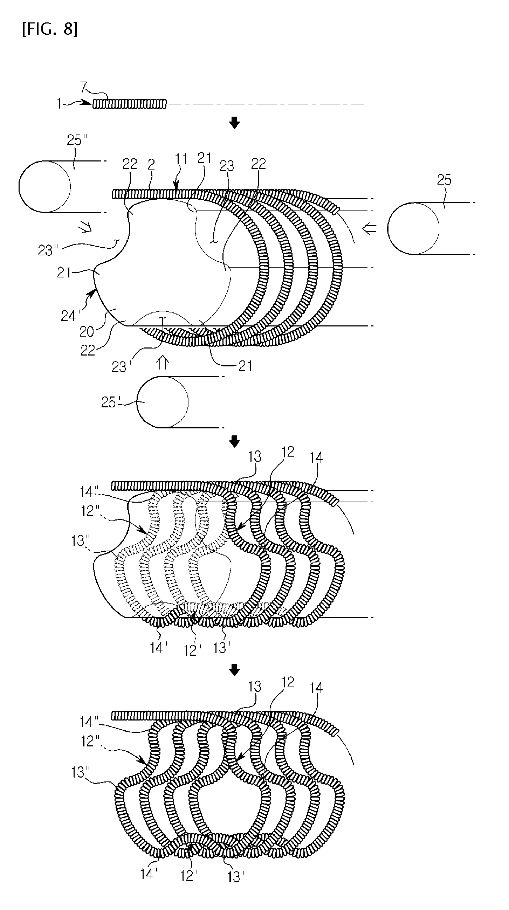

As shown in FIG. 8, this method of the invention uses a jig 24', which has curved end portions 21 and 22 and a plurality of curved recesses 23, 23' and 23" along the length of the circular rod 20. The curved end portions 21 and 22 face each other, and each of the curved recesses 23, 23' and 23" is connected to the curved end portions 21 and 22.

In the state in which the required length of the first coil 1 is wound on the jig 24', pressing circular rods 25, 25' and 25" are positioned at positions corresponding to the curved recesses 23, 23' and 23''. In this state, the first coil 1 is compressed in the lengthwise direction and is maintained in the compressed state.

In this state, the portions of the first coil 1 that are positioned in the curved recesses 23, 23' and 23" are inserted into the curved recesses 23, 23' and 23" by the respective pressing circular rods 25, 25' and 25''.

When the first coil is heat-treated such that it remembers this state, the embolus-forming in-vivo indwelling coil that the invention is intended to provide is realized. It is apparent that three curvature portions 12, 12' and 12" and six angle-changing portions 13, 14, 13', 14', 13'' and 14'' are formed in each of the unit helical coils 11.

Although it has been disclosed in the above description of the invention that one curvature portion 12 and three curvature portions 12, 12' and 12" are formed, this has merely been done for the sake of illustration and understanding of the invention. The right of the invention is by no means limited thereby. It is possible, of course, to form three or four curvature portions or more as required.

## Claims

1. An embolus-forming in-vivo indwelling coil in a helical coil (3), which is produced by preparing a helical first coil (1) by forming a wire (7) made of a material, such as platinum, silver, gold, palladium or tungsten, into a helical shape, heat-treating the first coil (1) so as to remember the helical shape, forming the first coil (1) into a helical shape again, and in this state, forming a secondary coil (2) by heat-treating the first coil (1) so as to remember this helical shape, **characterized in that** the secondary coil (2) comprises a plurality of unit helical coils (11), each of which has one or more curvature portions (12), both ends of each of the curvature portions (12) being integrally connected with angle-changing portions (13, 14), which are bent so as to be curved inwards.

2. The embolus-forming in-vivo indwelling coil of claim 1, wherein the curvature portions (12) and the angle-changing portions (13, 14) integrally connected with each of the curvature portions (12) are formed in positions on each of the unit helical coils (11) such that the curvature portions (12) face each other and the angle-changing portions (13, 14) face each other.

3. The embolus-forming in-vivo indwelling coil of claim 1, wherein a plurality of the curvature portions (12) and a plurality of the angle-changing portions (13, 14) that are integrally connected with each of the curvature portions (12) are formed on each of the unit helical coils (11) such that the curvature portions (12) have equal intervals from each other and the angle-changing portions (13, 14) have equal intervals from each other.

4. A method for manufacturing an embolus-forming in-vivo indwelling coil using a first coil (1), which is prepared by forming a wire (7) made of a material, such as platinum, silver, gold, palladium or tungsten, into a helical shape, and is heat-treated so as to remember the helical shape, **characterized by** a process of winding a required length of the first coil (1) on a jig (24), which has curved end portions (21, 22) and a curved recess (23) along a length of a circular rod (20), the curved end portions (21, 22) facing each other, and the curved recess (23) being connected to the curved end portions (21, 22); and a heat treatment process of heat-treating the first coil in a state in which predetermined portions of the first coil (1) that correspond to the curved recess (23) are pressed in a longitudinal direction using a pressing circular rod (25) while conducting the above process, so that a curvature portion (12) and angle-changing portions (13, 14) are formed in each of unit helical coils (11) of a secondary coil (2).

5. The method of claim 4, wherein three curvature portions (12, 12', 12'') and six angle-changing portions (13, 14, 13', 14', 13'', 14'') are formed in each of the unit helical coils (11) using a jig (24'), which has curved end portions (21, 22) and a plurality of curved recesses (23, 23', 23'') along the length of the circular rod (20), the curved end portions (21, 22) facing each other, and each of the curved recesses (23, 23', 23'') being connected to the curved end portions (21, 22).
